# EUROPEAN PATENT APPLICATION

(11) **EP 3 513 832 A1**
(43) Date of publication of application: **24.07.2019**
(21) Application number: 19152390.1
(22) Date of filing: 17.01.2019
(51) Int. Cl.: A61M 37/00

(54) **DEVICES FOR APPLYING LIQUID TO SKIN**

(30) Priority: 18.01.2018 CA 2992326
(71) Applicant: Xiao, Long, Scarborough ON M1P 5G8 (CA)
(72) Inventor: Xiao, Long, Scarborough ON M1P 5G8 (CA)
(74) Representative: Gualeni, Nadia

(57) **Abstract**

A needle assembly 10 for a liquid applicator includes a housing 40 comprising a tubular longitudinal channel 420 and a guide/inner surface 122. The channel comprises an upper open end and a lower open end. Liquid storage grooves 110 for storing liquid by capillary action extend generally transversely in the guide/inner surface above the lower open end. A needle bundle 200 is mounted in the housing. The needle bundle comprise one or more needles longitudinally reciprocally movable in the channel and being biased against the guide surface, or towards the inner surface, across the grooves such that, during longitudinal reciprocal movement of the needle bundle, the needle bundle maintains contact with the guide surface and the liquid stored in the grooves, or maintains a capillary gap between the needle bundle and the inner surface, for drawing the liquid out of the grooves by capillary action.

## Description

### FIELD

The present invention relates generally to devices for applying a liquid to skin, particularly to disposable needle assemblies utilizing capillary action for feeding ink or another liquid in such devices.

### BACKGROUND

A tattooing device typically includes a needle for applying ink to skin, a base with a needle actuator, and a needle handle that connects the needle to the base and can be conveniently held in a hand of an operator for manipulating the needle during use. Example tattooing devices have been described in, for example, US 6,505,530 to Adler et al. (January 14, 2003), US 8,454,643 to Crokett (June 4, 2013), CN204745346 to Zhao (November 11, 2015), and CN201595866 to Xiao (October 6, 2010), and US Patent Application Serial No. 15/691,125 by Xiao.

For safety reasons, the needle is typically sterilized before use and replaced after each use. It is thus convenient to use replaceable and disposable needle modules, which can be pre-sterilized and easily detached or connected to the needle handle so that the needle can be easily replaced, and safely disposed. Disposable needle modules, particularly single-use disposable needle modules or similar disposable needle assemblies are thus often used with tattooing devices and other similar devices.

Devices or applicators for applying permanent mark-up may have a similar construction, with a base, a replaceable handle, and a replaceable/disposable needle module.

US 6,505,530 disclosed a sterilized disposable module and a separate optional ink module. The ink module is attachable to an opening in the disposable module for filling an ink receiving portion in the disposable module. An ink tank or an ink cartridge may be provided. A micro dosing pump may be used to supply ink into the ink tank.

There are a number of the drawbacks in existing disposable needle assemblies. For example, some existing ink feeding techniques have not been widely adopted likely because they are inconvenient to use, or require complicated device structures and operation procedures.

US20130226211 by Xiao, published August 29, 2013, discloses a tattoo needle tip quipped with a capillary ink reservoir. A passage for mounting a tattoo needle is provided inside a needle tip body. A needle tip opening for the tattoo needle to protrude is provided at one end of the needle tip body. The needle tip body has an ink reservoir with a capillary ink chamber and an ink guiding passage for connecting the capillary ink chamber with the needle tip opening.

US20170072178 by Xiao, corresponding to CN 203898927 published on October 29, 2014, discloses a tattoo needle tip with a capillary ink reservoir. The tattoo needle tip includes a body and a needle passage in the body. An ink reservoir is directly connected to the needle passable by a capillary to directly feed ink to the sharp end of the tattoo needle.

It is still desirable to improve such and other devices for applying liquids to skin.

### SUMMARY

An aspect of the present disclosure relates to solving one or more problems present in the existing ink feeding techniques used for feeding ink in a tattooing device.

For example, it has been recognized that in some conventional capillary ink feeding techniques, even when there is still some ink in the ink reservoir, it often happens that little or no ink is supplied to the needle tip. Utilization of the ink in the ink reservoir is thus inefficient. Further, residual ink also makes it difficult to clean the ink reservoir and switch ink color during use. US20170072178 provides a better ink feeding technique, but further improvement is still desirable.

It has also been surprisingly discovered that ink feeding by capillary action may be improved by increasing direct contact between the needle shaft and the ink in the ink reservoir or the capillary ink feeding channel. Experiments have confirmed that a larger contact area provides a better feeding performance. Lack or limited contact between the needle shaft and the ink in the ink reservoir or feeding channel causes inconsistent ink flow and excessive residual ink.

Thus, in an aspect of the present disclosure, there is provided a needle assembly for a liquid applicator such as an ink applicator. The needle assembly includes a housing comprising a tubular longitudinal channel and an inner surface which may optionally serve as a guide surface. The channel has an upper open end and a lower open end. A plurality of liquid storage grooves for storing liquid therein by capillary action extend generally transversely in the inner surface above the lower open end. A needle bundle is mounted in the housing. The needle bundle includes one or more needles longitudinally reciprocally movable in the channel and is biased against or towards the inner surface, such as against the guide surface, across the grooves such that, during longitudinal reciprocal movement of the needle bundle, the needle bundle maintains contact with the liquid stored in the grooves for drawing the liquid out of the grooves by capillary action. During the longitudinal reciprocal movement of the needle bundle, the needle bundle may optionally maintain contact with the guide surface, or alternatively, a capillary gap between the needle bundle and the inner surface may be formed. The liquid may include ink.

Conveniently, the contact between the needle bundle and the liquid in the liquid storage grooves can be consistently maintained by the biasing force to provide stable and efficient liquid feeding.

The needle assembly may comprise a biasing member configured to bias the needle bundle to move longitudinally and against the guide surface. The biasing member may comprise a resilient band. The biasing member may comprise a silicone, latex, or rubber material. The guide surface may be shaped to conform to an external profile of the needle bundle to increase contact area between the needle bundle and the liquid stored in the grooves. The needle assembly may comprise a plurality of needles welded to one another. The needle bundle may comprise 1 to 27 needles. The housing may comprise a generally cylindrical or conical body having a circumference, and one or more of the grooves may extend up to 120° along the circumference of the body. The longitudinal channel may have a generally circular profile, or a generally polygonal profile. The grooves may have a width of about 0.3 mm to about 0.6 mm. The plurality of grooves may comprise 3 to 12 grooves. The housing may comprise a generally conical lower portion and a generally cylindrical upper portion. The grooves may be separated from one another. The grooves may be inter-connected. The housing may comprise an observation window opposite the guide surface, and the needle bundle may comprise a shaft portion and a needle tip portion, where the shaft portion has a first longitudinal axis and the needle tip portion has a second longitudinal axis, which is offset from the first axis towards the observation window.

In another aspect, there is provided a needle assembly for a liquid applicator, comprising a housing comprising a tubular longitudinal channel and an inner surface, the channel comprising an upper open end and a lower open end, a plurality of liquid storage grooves for storing liquid therein by capillary action extending generally transversely in the inner surface above the lower open end; a needle bundle mounted in the housing, the needle bundle comprising one or more needles longitudinally reciprocally movable in the longitudinal channel and being biased towards the inner surface across the grooves to form a capillary gap between the needle bundle and the inner surface for drawing liquid out of the liquid storage grooves during longitudinal reciprocal movement of the needle bundle across the grooves.

In a further aspect, there is provided a liquid applicator such as an ink applicator, which comprises a needle assembly disclosed herein. The liquid applicator may comprise a needle actuator for actuating downward movement of the needle bundle, and a handle coupling the needle actuator to the needle assembly. The liquid applicator may comprise a tattooing device.

Other aspects, features, and embodiments of the present disclosure will become apparent to those of ordinary skill in the art upon review of the following description of specific embodiments in conjunction with the accompanying figures.

### BRIEF DESCRIPTION OF THE DRAWINGS

In the figures, which illustrate, by way of example only, embodiments of the present disclosure:
Fig. 1 is a front elevation view of a disposable needle module, illustrative of an embodiment of the present disclosure;
FIG. 2 is an exploded perspective view of the needle module of FIG. 1;
FIG. 3A is a side cross-sectional view of the needle module of FIG. 1;
FIG. 3B is a side elevation view of certain parts of the needle module shown in FIG. 3A;
FIG. 3C is a force diagram showing the force applied to the needle bundle shown in FIG. 3B;
FIG. 4A is a partially-cut perspective view of the needle module of FIG. 1;
FIG. 4B is an axial cross-sectional view of the needle module of FIG. 4A, taken along line 4B-4B in FIG. 4A;
FIG. 4C is an enlarged view of the portion 4C of the needle module of FIG. 4A;
FIG. 4D is an enlarged view of the portion 4D of the needle module of FIG. 4B;
FIG. 5A, 5B, 5C, 5D and 5E are axial cross-sectional views of various variants of the needle module, illustrative different embodiments of the present disclosure;
Fig. 6 is a side elevation view of a tattooing device, with partial cut-away cross-sectional view of the needle handle and needle module in the tattooing device, illustrative embodiments of the present disclosure;
FIGS. 7A and 7B are exploded and perspective views of the needle in the needle module of FIGS. 1 and 2, respectively, illustrating the assembly of the needle;
FIGS. 8A and 8B are perspective views of the cap in the needle module of FIGS. 1 and 2;
FIG. 9A is a perspective view of another needle module, according to an embodiment of the present disclosure;
FIG. 9B is a side elevation view of the needle module of FIG. 9A;
FIG. 9C is an enlarged cross sectional view taken along the line 9C-9C of FIG. 9A.

### DETAILED DESCRIPTION

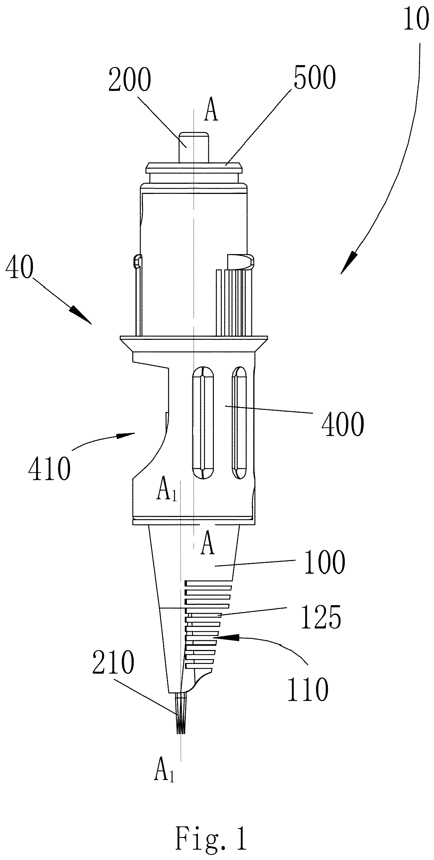

An embodiment of the present disclosure relates to a needle assembly, such as needle module 10 as illustrated in FIGS. 1 to 4C.

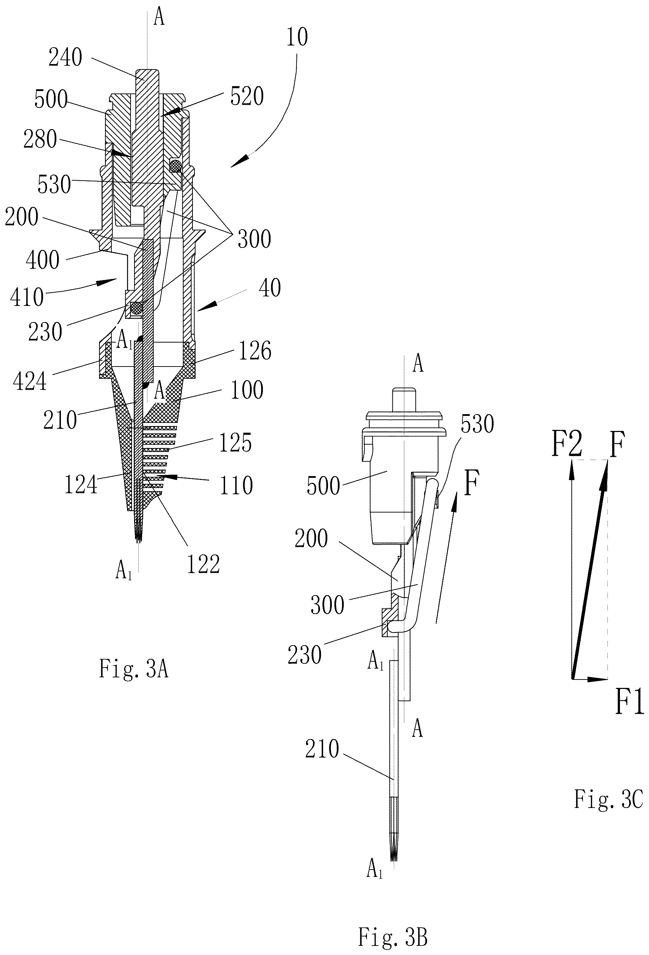

As depicted in FIGS. 1 to 3A, needle module 10 includes a needle housing 40 for movably mounting a needle bundle 200 therein. The needle housing 40 is formed by a tubular body portion 400, a mouthpiece 100 and a cap 500. Housing 40 has a tubular longitudinal channel 420 therein. The channel 420 has an upper open end 422 and a lower open end 424. A mouthpiece 100 with an opening 120 is provided at the lower end 424 and a cap 500 is provided at the upper end 422. Mouthpiece 100 has a guide surface 122 (see FIG. 3A) for guiding movement of the needle and feeding ink to the needle, as will be detailed herein below. The channel 420 may be formed with a tubular body portion 400, which may have an observation window 410. Channel 420 has a longitudinal axis denoted as axis A in the figures. The opening 120 in the mouthpiece 100 has a longitudinal axis denoted as axis A₁ in the figures.

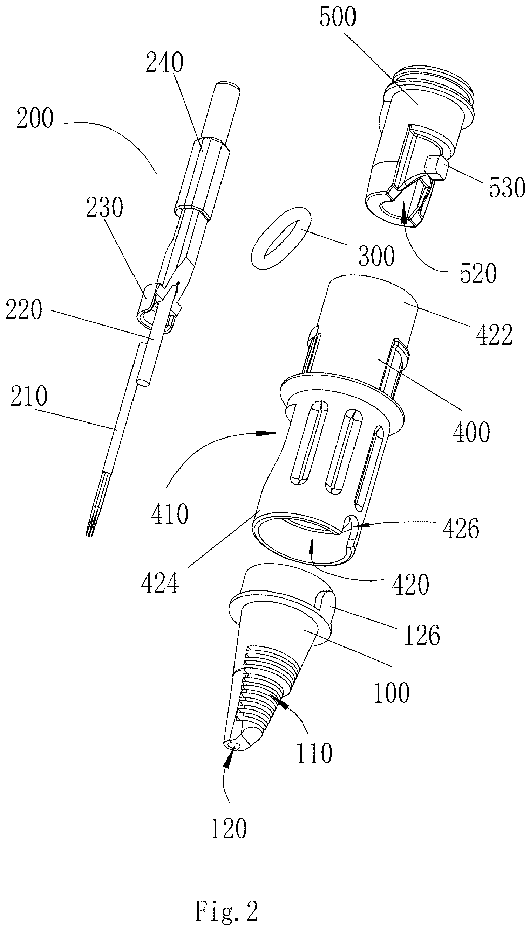

Mouthpiece 100 and cap 500 may be connected and engaged with the lower and upper ends of body portion 400 in any suitable manner, with any suitable engagement or locking mechanism. For example, these parts may be engaged by tabs, threads, clamps, pins, keys, and corresponding openings, notches, threads, holes, keyways, or the like as can be understood by those skilled in the art. As depicted, both mouthpiece 100 and cap 500 may frictionally engage the inner walls of body portion 400, and are interlocked in the mounted positions with a tab. For example, as illustrated in FIG. 2, the housing body portion 400 may include a mating notch 426 at the lower end 424, and the mouthpiece 100 may include a corresponding mating stab 126 that matches and mates with the mating notch 426 for orienting and positioning the mouthpiece 100 in relation to the housing body portion 400.

As will be appreciated and as in conventional needle modules, needle bundle 200 is housed and guided in housing 40 in a manner to allow the needle bundle 200 to reciprocally move up and down during use. The detailed construction and mechanism for such mounting and reciprocal movement are not the focus of this disclosure, and can be implemented by a skilled person in the art according to known techniques or constructions, except in aspects specifically described below. Thus, some of these details will not be discussed herein.

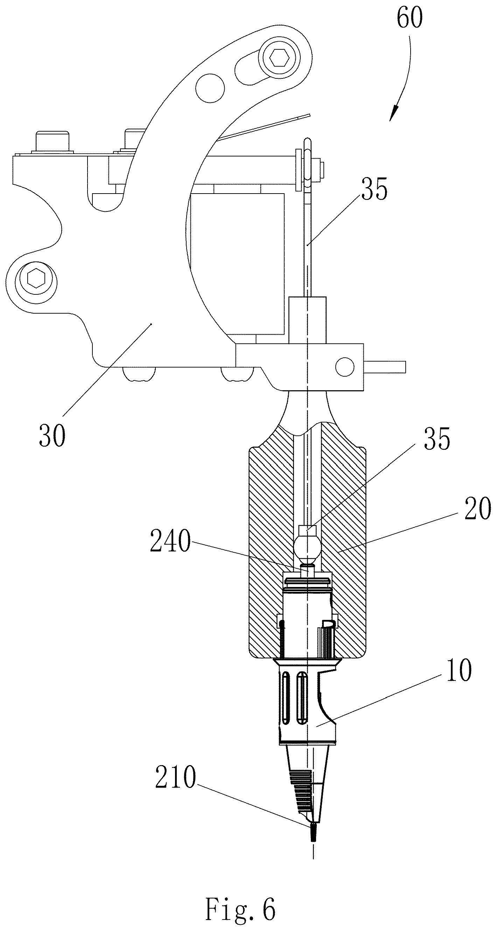

It is noted that, however, to facilitate the reciprocal movement of the needle bundle 200, a biasing member such as a resilient band 300 is also provided and configured to pull the needle bundle 200 up during each movement cycle after the needle bundle 200 is pushed down by an actuating or driving mechanism such as a drive shaft (not shown but see FIG. 6 to be discussed later). In an embodiment in the present enclosure, the biasing member such as band 300 is not only used to facilitate reciprocal movement of the needle bundle 200, but also used to bias the needle bundle to increase or maintain the contact between the needle bundle 200 and the stored ink in the ink reservoir as will be made clearer below.

As will be understood, needle module 10 can also be considered as a needle assembly. While the depicted device is referred to as a needle module, it is only used to illustrate an embodiment of possible needle assemblies. In different embodiments, an embodiment of the present disclosure may be used in any number of different types of needle assemblies.

Returning to FIGS. 1 and 2, as depicted, needle bundle 200 includes a tip portion 210, a needle shaft portion 220, a hook structure 230, and a shaft 240.

Tip portion 210 may include one or more needle tips, which may be welded together, or otherwise bounded together. The needle tips may be formed of stainless steel or any other suitable material. The individual needle tip may have any suitable or known needle tip shape. The needle tips may be arranged to form a tip portion that has a generally or substantially cylindrical or conical profile. Alternatively, the needle tips may be arranged side-by-side to form a tip portion that has a generally flattened or band-shaped profile. Rows of side-by-side needles may also be stacked. Such different arrangements of tattoo needles are known in the art and may be referred to as "Round Liner" needles, "Round Shader" needles, "Flat" Needles, or "Magnum" needles, respectively. Tip portion 210 may include 1-18 individual needle tips for "Round Liner" needles and "Round Shader" needles, or may include 4-27 needle tips for "Flat" needles and "Magnum" needles.

As can be appreciated, the cross-sectional sizes or diameters of the needles or needle bundles will affect how the ink will flow. Typically, the smaller the needle tip size or narrower the diameter of the needle tip, the finer and more controlled the stream of ink that flows off each needle tip. Typically, the needle tips in the same needle bundle may be of the same or similar sizes. The size of the needle tips may be selected based on the desired effects by the operator or user. Different sizes may be used for different reasons. Standard sizes of needles may be used. The diameters of the individual needles may be 0.25 mm, 0.30 mm, or 0.35 mm in some embodiments. The designs of the needle tips may be selected and vary as known in the art based on the desired tattooing techniques and purposes to be applied.

The number of needles in a needle bundle may vary from 1 to 27 or more as desired. For example, commercially available round needle bundles typically have 1, 3, 5, 7, 8, 9, 11, 14, or 18 needles in each bundle. It would also be appreciated that the overall profile of the needle bundle may change and vary depending on the number of needles in the bundle, their arrangement, the amount of soldering material used, or other factors.

The needles in a bundle may be welded together, such as by lead-free soldering.

As can be seen in FIG. 2, FIGS. 3A and 3B, and FIGS. 7A and 7B, the welded needle tips in tip portion 210 are supported on needle shaft portion 220. The axis of needle shaft portion 220 and the axis of shaft 240 are aligned with the axis A of the channel 420 in the housing body portion 400, but are off-set from axis A₁ of the opening 120 of the mouthpiece 100 and the axis of needle tip portion 210. The needle shaft portion 220 may be formed of stainless steel or another suitable material. It should be sufficiently rigid and strong to support stable movement of the needle tip portion 210 during operation. Tip portion 210 may be welded onto needle shaft portion 220.

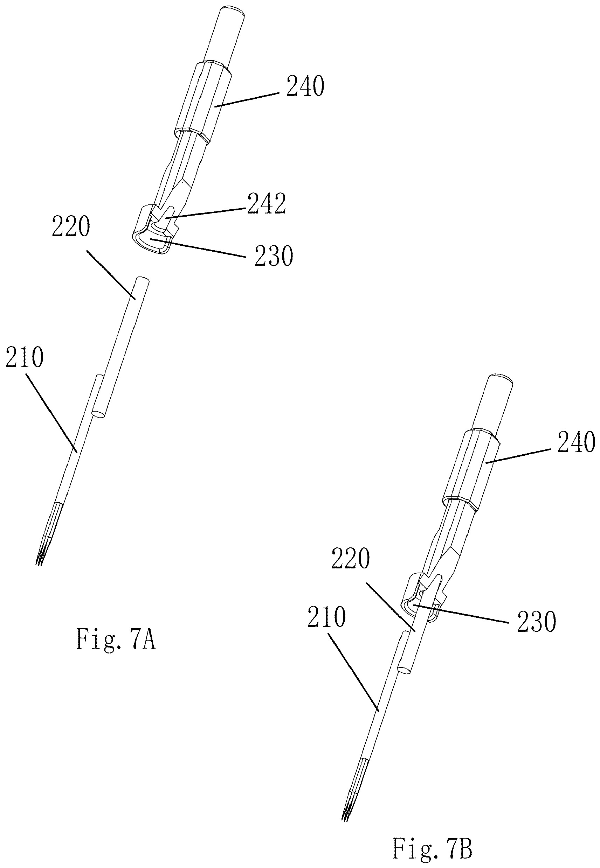

Needle shaft portion 220 is joined with shaft 240 for driving the tip portion 210. Shaft 240 may be formed of a plastic material or another suitable material for transmitting the axial driving force to needle shaft portion 220 and then indirectly to tip portion 210. As depicted in FIGS. 7A and 7B, shaft 240 may include a cylindrical bore 242 for receiving the needle shaft portion 220 at the lower end of the shaft 240. Needle shaft portion 220 may be inserted into the bore 242 and may be attached to the wall of the bore 242 of the shaft 240 with an adhesive, such as glue. At the bottom end of the shaft 240, a hood-shaped hook 230 is provided adjacent to the bore opening of bore 242. The hook 230 is shaped and sized to accommodate and retain a section of the elastic band 300, which is looped around needle shaft portion 220 during assembly and is retained between needle shaft portion 220 and hook 230. As can be appreciated, other forms or structures may also be used to retain band 300. The hood-shaped hook 230 provides a secure retaining mechanism for retaining the band 300 when needle bundle 200 moves up and down, as it prevents accidental release of the band section over the hook 230 due to fast relative movement of the parts.

Hook 230 may be replaced by a hook mounted on shaft 240 in a different embodiment.

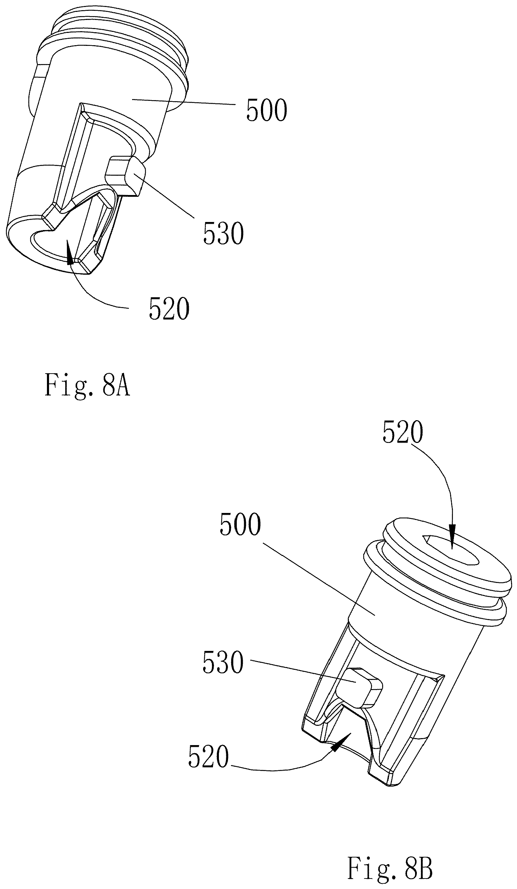

The cap 500 may be made of a plastic material, or any other suitable material. As better seen in FIGS. 8A and 8B, cap 500 has a through opening 520 that allows the shaft 240 to axially move therethrough. A stub 530 is provided on cap 500, which stub faces the inner wall of in the body portion 400 of the housing 40.

As illustrated in FIGS. 3A and 3B, a section of the band 300 may be looped around the stub 530 on the cap 500, and retained between the stub 530 and the inner wall of the body portion 400 of the housing 40.

An elastic looping band 300 is provided, which, when the needle module 10 is assembled, is looped around at one end hook 230 and at the other end around stub 530, so that band 300 is hooked by hook 230 and stub 530 so that band 300 is retained in place but can expand and contract when the needle bundle 200 moves up and down during operation. The hook 230 and stub 530 are configured and oriented during assembly such that when band 300 engages both hook 230 and stub 530, band 300 biases the needle bundle 200 upward and biases the tip portion 210 against the guide surface 122 in the mouthpiece 100. Band 300 may be formed of a silicone band, or a band formed of another resilient material such as latex or rubber. Band 300 may be in the shape of an O-ring when in a relaxed state.

As better illustrated in FIGS. 3A to 3C, the band 300 is normally positioned and oriented at a slight angle relative to the axial direction of shaft 240, in the F direction as shown in FIG. 3B. For easier viewing, the mouthpiece 100 and body portion 400 are not shown in FIG. 3B. The band 300 is normally slightly tensioned to bias the needle bundle 200 upward (i.e. to retract) and bias the tip portion 210 against the guide surface 122. When the needle bundle 200 is pushed downward by a drive shaft (not shown) through shaft 240, the band 300 is further stretched and provides an increased axial biasing force upward (component F2 as illustrated in FIG. 3C). However, the radial component of the biasing force (F1 shown in FIG. 3C) against the guide surface 122 will not change significantly during axial movement of the needle bundle.

As can be appreciated, the inner diameter of the through opening 520 in cap 500 may be sized to be larger than the outer diameter of the shaft 240, and there may be gap 280 between the shaft 240 and the inner wall that defines opening 520. Such a gap may allow smooth movement of the needle bundle 200 and shaft 240 in the housing 40, reducing or preventing the risk of jamming or too much friction. Such a gap also allows the shaft 240 and needle bundle 200 to lean at a slight angle off the axial direction (axis-A).

Under the biasing force (denoted by F1 as shown in FIG. 3C) exerted by the band 300, the tip portion 210 at the bottom end of needle bundle 200 is pressed towards guide surface 122. A slight lean of needle bundle 200 off the axial direction of the axis A or A₁ would reduce the stress and bending of needle bundle 200.

As depicted, the axis A₁ of opening 120 and the needle tip portion 210 is offset from the axis A of the shaft 240 and needle shaft portion 220. The mouthpiece 100, needle shaft portion 220, shaft 240, body portion 400 and cap 500 may be co-axial in some embodiments, as depicted in FIG. 3A (aligned along axis A). As depicted in FIGS. 1 and 3A, the axis A₁ is offset from the axis A towards the observation window 410, so that the axis A₁ is closer to the observation window 410 than the axis A. The distance between axis A and A₁ may be from about 1.2 mm to about 1.8 mm. The needle module 10 is typically held by the operator so that the observation window 410 is facing the operator during operation. As depicted, when the needle module 10 is properly assembled, the observation window 410 and the guide surface 122 are on opposite sides of the axis A₁.

In different embodiments, the shaft 240 and needle shaft portion 220 may also be offset and non-coaxial. In such cases, the offset distance between the axis A₁ and the axis A may be even larger, such as about 2 mm to about 3 mm, or up to 5 mm.

The mouthpiece 100 is configured to support and allow axial movement of the needle tip portion 210 through the opening 120. The mouthpiece 100 may be made of a plastic material or another suitable material. The opening 120 may have a shape configured to match the profile of the tip portion 210 of needle bundle 200. For example, for round or circular tip portion 210, the opening 120 may have a circular or diamond shape; for flat tip portion 210, the opening 120 may have a rectangular shape.

As depicted in the drawings, the mouthpiece 100 and housing body portion 400 may be generally co-axial (along axis A) but the axis A₁ of the opening 120 may be offset from axis A of channel 420. As can be appreciated, when the axis A₁ of the opening 120 is offset from the axis A of the channel 420, the needle tip portion 210 is also offset from axis A to align with the opening 120. Conveniently, the embodiment as depicted allows the operator to better see the position of the sharp needle tip(s) during operation. To allow improved observation, mouthpiece 100 may be made of a transparent material, such as a transparent plastic. The offset of the opening 120 with respect to the channel 420 also provides more space to accommodate larger ink storage grooves 110, thus providing higher storage capacity.

The size of opening 120 is also selected to accommodate the size of the tip portion 210. To avoid jamming during use and to accommodate different needle sizes or needle bundle sizes, the size of the opening 120 of the mouthpiece 100 may be selected to allow some play of the largest needle bundle to be used. That is, the size of opening 120 is slightly larger than the size of the needle tip portion 210, so that there is a gap between the inner wall of opening 120 and the needle tip portion 210 on the opposite side of the guide surface 122. This gap allows smooth movement of the needle tip and size variations of the tip portion 210, without jamming.

Even though there is a gap between the needle tip portion 210 and the opening 120, the risk that the tip portion 210 will vibrate sideways is low as the tip portion 210 is biased against the guide surface 122, which provides a stable support surface for the movement of the needle tip. In other words, even when the size of the opening 120 is relatively large, and there is a gap between the tip portion 210 of the needle bundle 200 and the inner surfaces of the mouthpiece 100, the biasing force F1 causes the tip portion 210 to contact and abut against the guide surface 122, and maintain the contact during operation (reciprocal movement of the needle bundle 200).

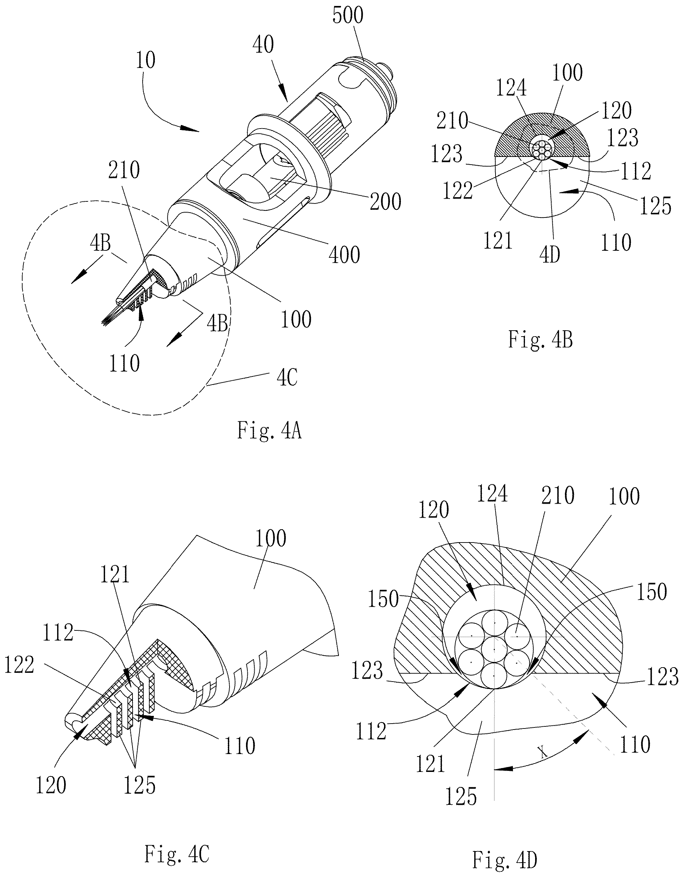

Mouthpiece 100 is also configured to function as an ink feeding device. Specifically, as illustrated in FIG. 3A, and in FIGS. 4A to 4D, ink storage grooves 110 defined by groove walls 125 are provided on guide surface 122, for storing ink. Each groove 110 extends generally transversely in relation to the axial direction and the direction of movement of the needle bundle 200. Groove walls 125 and grooves 110 may be generally parallel to one another, and are located near, but above, the lower open end of mouthpiece 100. Grooves 110 may extend radially to the external surface of the mouthpiece 100 to allow easy filling of ink during use. That is, each groove 110 may be open to the external surface of mouthpiece 100 as depicted in these figures. Each individual groove 110 is formed between two opposing groove walls 125 having a thin gap between the opposing groove walls 125 such that when the mouthpiece 100 is brought into contact with an ink source (such as when it is dipped in an ink bottle), ink can be sucked into and fill the gap due to capillary action. The ink in the gap can be normally retained in place due to surface tension and adhesion of the ink liquid to the groove walls 125.

However, when the tip portion 210 of the needle bundle 200 moves downward across the grooves 110 while being pressed against the guide surface 122, the moving needles will contact the liquid surface of the ink stored in the grooves 110 and bring ink out of grooves 110, and then carry the ink with the needles or allow the ink to flow along the needles and be applied to the skin. Close contact with the guide surface 122 also facilitates flow of the ink into narrow gaps 150 (see FIG. 4D) that exist between the needles in the needle bundles 200 and the guide surface 122.

For example, as better illustrated in FIGS. 4B and 4D (also see FIG. 4C), in an example bundle of seven (7) needles, it is possible that only the bottom needle is in contact with the guide surface 122, at a point 121. A section of the bottom needle may be in contact with the guide surface 122 between the grooves 110, and may extend across the grooves in a direction that is particular to the traversal directions along which the grooves 110 extend. However, narrow gaps 150 also exist between the curved guide surface 122 and some of the needles in tip portion 210 of the needle bundle 200. A much larger gap is present between the tip portion 210 and the opposite wall surface 124 of the inner channel of the mouthpiece 100 as depicted in FIG. 4B (also see FIG. 4D).

The grooves 110 are also open at the guide surface 122, with ink contacting openings 112. The width of each ink contacting opening 112 may be about 0.3 mm to 0.6 mm. The width may vary and may be selected based on the ink fluid properties. In a particular embodiment, the width of opening 112 may be about 0.4 mm.

The number of grooves 110 may also vary and may be selected based on a number of factors, such as the amount of ink to be applied and the size and length of the needles used, and production or manufacturing considerations. In some embodiments, there may be 3 to 12 grooves, such as 5 to 10 grooves.

The contact opening 112 of each groove 110 at the guide surface 122 may extend circumferentially by a selected angle θ around the axial direction (see axis A₁ in FIG. 1). The contact opening 112 may extend symmetrically about the contact point 121, by an angle of X on each side (see FIG. 4D), where θ = 2X. In different embodiments, angle θ may vary from about 120° to about 360°. For example, angle θ may be about 120°, 140°, 160°, 180°, or 200°.

The external openings of grooves 110 may extend circumferentially at similar angles or larger angles. As depicted in FIGS. 4B and 4D, in an embodiment, the external openings of grooves 110 may extend continuously, and may be limited/defined by a wall 123 that extends along the line between the ends of the contact opening 112.

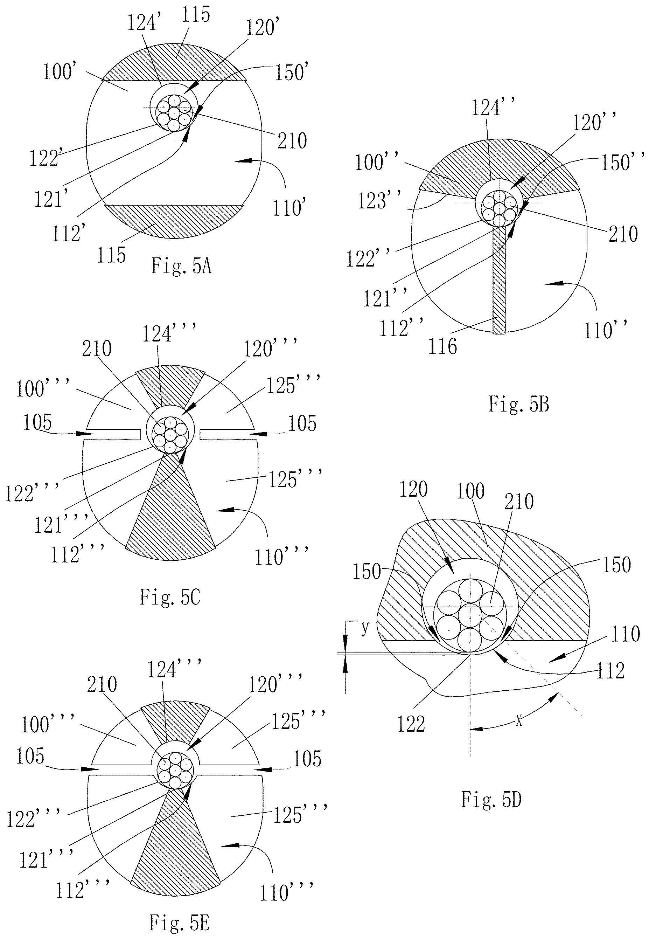

In alternative embodiments, the external openings of grooves 110 may be segmented, as illustrated in FIGS. 5A, 5B and 5C.

FIG. 5A illustrates a portion of a variant of the mouthpiece 100' having ink storage groove 110' with contact opening 112', the angle θ is 360°, an opening 120', a guide surface 122', and an opposite surface 124'. Needle bundle tip portion 210 is received in opening 120', and contacts the guide surface 122' at contact point 121'. There is a narrow gap 150' between tip portion 210 and the guide surface 122' around the contact point 121'. In FIG. 5A, the external opening is separated by solid sections 115. Sections 115 provided at opposite sides can provide reinforcement of the structural integrity. It is possible to have a section 115 at only one side, in which case, however, the mechanical strength of the structure may be reduced and the mouthpiece 100 may be easier to bend or deform.

FIG. 5B illustrates a portion of a variant of the mouthpiece 100" having ink storage groove 110" with contact opening 112", an opening 120", a guide surface 122", and an opposite surface 124". Needle bundle tip portion 210 is received in opening 120", and contacts the guide surface 122" at contact point 121". There is a narrow gap 150" between tip portion 210 and the guide surface 122" around the contact point 121" and at contact opening 112". In FIG. 5B, the external opening is separated by a reinforcing section 116, which can increase the structural strength and prevent or reduce bending or deformation.

FIG. 5C illustrates a portion of a variant of the mouthpiece 100"' having ink storage groove 110'" with contact opening 112"', an opening 120"', a guide surface 122"', and an opposite surface 124"'. Needle bundle tip portion 210 is received in opening 120"', and contacts the guide surface 122"' at contact point 121'". There is a narrow gap 150"' between tip portion 210 and the guide surface 122"' around the contact point 121"' and at contact opening 112"'.

In FIG. 5C, fluid conduits 105 are provided on groove walls 125'" to allow fluid communication between different grooves 110"'. Conduit 105 thus connects grooves 110"'. The width of conduit 105 is smaller than the width of the groove 110"'. For instance, if the groove 110"' has a width of 0.4 mm, conduit 105 may have a width of about 0.3 mm.

In other embodiments, conduit 105 may be deeper or shallower, and may even extend all the way to the guide surface 122'" so that ink may be fed directly to tip portion 210, as illustrated in FIG. 5E.

As can be appreciated, section 115 or 116 may provide a stronger construction and stabilize the groove walls 125, and thus the gaps therebetween, although providing section 115 or 116 may reduce the ink storage volume in the grooves 110.

In the embodiment illustrated in FIG. 5B, the bottom needle or the tip portion 210 of the needle bundle 200 may be in continuous contact with the guide surface 122" at contact point 121". Section 116 may be formed of a reinforcing material to improve the mechanical strength of the mouthpiece. The guide surface at the section 116 may also be smooth, and may optionally be provided with special surface treatment for reduced friction between the needle(s) and the guide surface 122".

With the added section 115, or 116, it is also possible to further extend the circumferential angle of each groove 110' or 110", as illustrated in FIGS. 5A and 5B respectively, such as to an angle higher than 180° or is 360°. Groove wall 123" may also extend at an angle relative to the line between the ends of the contact opening 112", to allow larger external openings for easier ink filling and increased ink storage capacity.

As illustrated in FIG. 5D, it is possible in some embodiments that some portions of the bottom needle are not in contact with the guide surface 122, but is very close to the guide surface 122 due to the biasing force (F1), separated by a small distance "y". As long as the gap y is small enough, ink may still be drawn from ink storage grooves 110. A benefit of no-contact between the needle and the guide surface 122 is that there is reduced or no friction.

Needle module 10 may be pre-sterilized and packaged to keep it clean during storage and transportation.

The needle module 10 and its parts may be manufactured from suitable materials based on technologies known to those skilled in the art. For example, various parts in the needle module 10 may be made from corresponding materials used in conventional needle modules, such as those used in tattooing needle modules. The parts may be formed and machined using known processing techniques, as can be understood by those skilled in the art in view of the present disclosure.

During use, needle module 10 is installed on a corresponding tattooing device. For example, as illustrated in FIG. 6, needle module 10 may be coupled, through or by a needle handle 20, to a base device 30.

The base device 30 may typically include a needle actuator with a drive shaft 35 for actuating and driving the needle tip portion 210 through shaft 240.

The handle 20 may have a generally tubular shape, and may be cylindrical.

The handle 20 and base device 30 may be constructed and operated as disclosed in US Patent Application Serial No. 15/691,125 by Xiao, the entire contents of which are incorporated herein by reference.

The assembled needle module 10, handle 20, and base device 30 form an ink applicator 60, or in particular, a tattooing device.

During operation, an operator may hold the ink applicator 60 with handle 20 in her hand, and dip the mouthpiece 100 in an ink container (not shown) to fill the ink storage grooves 110 with the desired ink. The needle actuator is activated to drive the drive shaft 35 and consequently needle shaft 240 and tip portion 210 of the needle bundle 200 downward. After each downward stroke, the resilient band 300 is stretched and pulls the tip portion 210 back up once the drive shaft 35 stops the downward movement and moves upward. The needle actuator then re-starts the downward drive in the next cycle. This process repeats so as to drive the needle bundle 200 to reciprocally move longitudinally along the axial direction of the shafts 35 and 240. The base device 30 may be configured to operate at a drive frequency of about 80 to 150 Hz. The operator may adjust the operating frequency during operation. In some tattooing machines, the tattoo needles may be operated to puncture the skin from 50 to 10,000 times per minute. The needles may penetrate the skin and reach a depth of about 1 mm. The vertical moving distance of the drive shaft 35 is typically about 2 mm to 5 mm, and the needle bundle 200 may similarly move about 2 to 5 mm during each stroke.

In different embodiments, drive shaft 35 may be connected to needle bundle 200 through a coupling member (not shown). During a stroke, drive shaft 35 may push needle bundle 200 downward, and thus stretch the band 300. After reaching the bottom of the stroke, the drive shaft 35 and band 300 will both pull needle bundle 200 back up.

As the tip portion 210 moves up and down, the operator may bring it to contact a subject's skin to apply ink to the skin, as in a conventional operation, which can be understood by those skilled in the art.

As discussed above, due to the biasing force (F1) applied by band 300, tip portion 210 is biased against the guide surface 122 in mouthpiece 100 while moving up and down due to the longitudinal reciprocal movement, and can draw ink out of ink storage grooves 110.

After each operation or use, the needle module 10 may be removed and disposed. The handle 20 may be next removed, and may also be disposed. The operator can take off disposable gloves and clean her hands at this time before touching other parts of the ink applicator 60.

For the next operation, a new needle module and new handle may be connected to the base device 30, and used similarly as described above.

The needle module 10 may be used directly after opening the needle module packaging without further cleaning, sanitization, or sterilization, and can be disposed after a single use without cleaning or any other treatment.

Conveniently, ink flow from grooves 110 to the needle tips may be stable and consistent, when there is still sufficient ink in grooves 110. Ink residue in the grooves 110 before re-fill can be reduced, as compared to some conventional ink feeding techniques. More efficient use of the ink and ink fill is thus possible.

As the contact openings 112 of grooves 110 extend perpendicular to the axial direction of tip portion 210 and its movement direction, it is unlikely the tip portion 210 may stuck in a groove 110. In comparison, if the ink storage groove is parallel to the needle movement direction, the needle may be more likely to get stuck in the groove.

Further, a tattoo operator tends to hold the needle in a relatively vertical orientation or an inclined orientation close to the vertical orientation. When the grooves run parallel to the needle movement direction, i.e., vertical or nearly vertical during operation, the ink in the groove may tend to flow downward due to gravity. To prevent such undesired ink flow, the vertical grooves will need to be narrower to limit the effect of gravity. In comparison, as the groove walls 125 between the grooves 110 are placed horizontally or close to horizontal during operation, gravity will have less effect on the fluid flow in the grooves 110. As a result, relatively larger grooves (or gaps between groove walls) may be used for ink feeding in an embodiment of the present disclosure, and consequently, the amount of ink stored during each fill or re-fill is increased as compared to some conventional ink feeding devices.

With perpendicular grooves, the number of grooves may also be varied, and increased as compared to vertical grooves. With vertical grooves, the number of vertical grooves is limited due to the limited diameter of the needle size (or size of the needle bundle). Tests have shown that with vertical grooves, while the grooves directly in contact with the needle tended to allow excessive ink flow, the grooves not in direct contact with the needle tended to keep excessive residual ink even when the needle was no longer delivering ink to the skin. That is, vertical grooves further away from the needle are not very effective for feeding ink. Such limitation and problems do not exist with particular grooves in an embodiment disclosed in the present disclosure.

It might have been expected that perpendicular groove orientation is not as effective as the needle would need to travel across groove edges, which might have been expected to cut off ink flow, so that it might have been expected that perpendicular groove orientation should not be used. However, tests have surprisingly shown that example embodiments as disclosed herein could deliver and feed ink more stably and consistently, and could address a number of drawbacks of conventional feeding techniques discussed herein.

For example, without being limited to a particular theory, it is expected that, while the ink flow from grooves 110 in a vertically held needle bundle 200 may be mainly due to capillary action (gravity effect is limited by the horizontal groove walls 125 as discussed above), since the small gaps 150 between the guide surface 122 and needle tip portion 210 may be smaller than the groove width in grooves 110, the capillary force in the gaps 150 may be larger than the capillary force in the grooves 110. As such, ink will likely be drawn out of the grooves into the small gaps 150 due to such force differential. Ink residual in the grooves 110 may be reduced as a result.

Another convenient effect of an embodiment disclosed herein is that the size of the opening 120 in the mouthpiece 100 may be adjusted with more flexibility. Due to the biasing force applied to the tip portion 210 by the band 300, contact and alignment of the tip portion 210 with guide surface 122 may be more conveniently maintained without a close-fit opening 120. With a larger opening 120, it is possible to reduce friction between the needle tip portion 210 and the wall surface at opening 120.

For clarity, it is noted that "single use" may refer to use of a needle or needle module for one complete operation on a single individual subject. During this operation, different needle modules may be used to apply different ink colors or for different purposes. For example, it may be typical to use two to five different types of needles during a single operation on a subject, depending on the complexity of the design to be applied.

As can be appreciated, various modifications may be made to the example devices illustrated in the drawings.

For example, band 300 may be formed of any suitable material with suitable strength and resiliency. Further, the O-ring and hook structure may be replaced with another biasing mechanism or structure. Other biasing members known to skilled persons in the art may also be used as long as they provide the desired biasing force to draw back the needle tip and bias the tip portion against the guide surface 122. In some embodiments, separate biasing members may be used to separately provide the longitudinal biasing force F2 and the radial biasing force F1 (see FIG. 3C). However, as can be appreciated, band 300 conveniently provides both biasing forces with a simple construction structure. Alternative biasing members may include elastic bands, springs, resilient stabs, or the like. Suitable resilient materials may include silicones, rubbers, latex, plastics, metals, or the like.

To further reduce friction and possible adverse edge effects at the edges of contact openings 112, the edges at openings 112 may be treated for smooth contact, and may be rounded to eliminate sharp edges. As described above, directed contact maybe limited or reduced to a small contact point. However, when the needle bundle does not directly contact the guide surface, the distance between the needle and the guide surface should be relatively small, such as below 0.15 mm, to allow the needle to draw ink out of the grooves 110. As noted above, a further alternative to avoid or limit edge effect, is to provide a smooth guide surface at the contact point, such as illustrated in FIG. 5B and FIG. 5C.

In some embodiments, the groove walls 125 in grooves 110 may be provided with cavities or holes (not shown) to store more ink. Ink storage grooves may include smaller segments of openings arranged in rows and columns. Such openings may have circular, rectangular, oval or other profiles or shapes. The arrangement of the openings and grooves may be regular or irregular. The tip portion 210 may be biased toward and to move across the arrangement of the openings so as to draw out ink by capillary action.

As the amount of stored ink may be increased, while reducing the risk of excessive residual ink or unstable ink flow, an embodiment of the needle assembly disclosed herein may allow an operator to fill the ink less frequently, and reduce the operation time.

As now can be appreciated, when guide surface in the mouthpiece is shaped to conform to an external profile of the needle bundle, the contact area between the needle bundle and the ink stored in the grooves can be increased.

As described herein, the circumferential angle refers to the angle formed by two lines from the center of a circle to two points on the circumference of the circle. It should be noted that, however, in practice, the parts in the disclosed devices may not have perfect circular shapes, and the profiles of the parts may be generally circular and the angles may be approximate.

The longitudinal channel in the housing may be considered to have a generally circular profile even if the profile is not a perfect circle. In some embodiments, the longitudinal channel may have a generally polygonal profile, which is not necessarily perfect polygon. For example, the profile may be generally rectangular or a diamond shape, but the edges may be no-linear.

In some embodiments, the ink storage grooves may have a width of about 0.3 mm to about 0.6 mm, but in other embodiments the width may be wider or narrower.

As depicted in the figures, the housing in the needle module may include a generally conical lower portion (mouthpiece) and a generally cylindrical upper portion (body portion). However, in different embodiments, the housing and any of its components may have different shapes and sizes.

The ink storage grooves may be separated and isolated by groove walls, or may be inter-connected via channels or holes through the groove walls. The groove walls may also be perforated. The connecting channels and holes may be sized for capillary action, or may have relatively larger sizes as it is not necessary that the fluid communication between the grooves through the groove walls are effected by capillary action. Interconnected grooves may allow faster and more uniform ink loading and distribution along the needle length, and hence more effective ink feeding.

As described earlier, in an embodiment, the tip portion of the needle bundle may not contact the inner surface of the mouthpiece during operation. In such cases, the housing may include a tubular longitudinal channel and an inner surface. The channel has an upper open end and a lower open end, a plurality of ink storage grooves for storing ink by capillary action extending generally transversely in the inner surface above the lower open end. A needle bundle is mounted in the housing, which includes one or more needles longitudinally reciprocally movable in the longitudinal channel and are biased towards the inner surface across the grooves to form a capillary gap (such as the gap with gap distance y as illustrated in FIG. 5D) between the needle bundle and the inner surface, for drawing ink out of the ink storage grooves during longitudinal reciprocal movement of the needle bundle across the grooves.

In some embodiments, the ink storage grooves may be closed to the exterior surface of the mouthpiece, and an ink container may be provided and placed in fluid communication with the grooves, such as via an internal fluid conduit though the groove walls. The ink container may be external to the needle module, in which case, the ink container may be connected to the internal fluid conduit by a tube. The ink container may also be installed within the housing in the needle module, or attached to the handle or another part of the ink applicator.

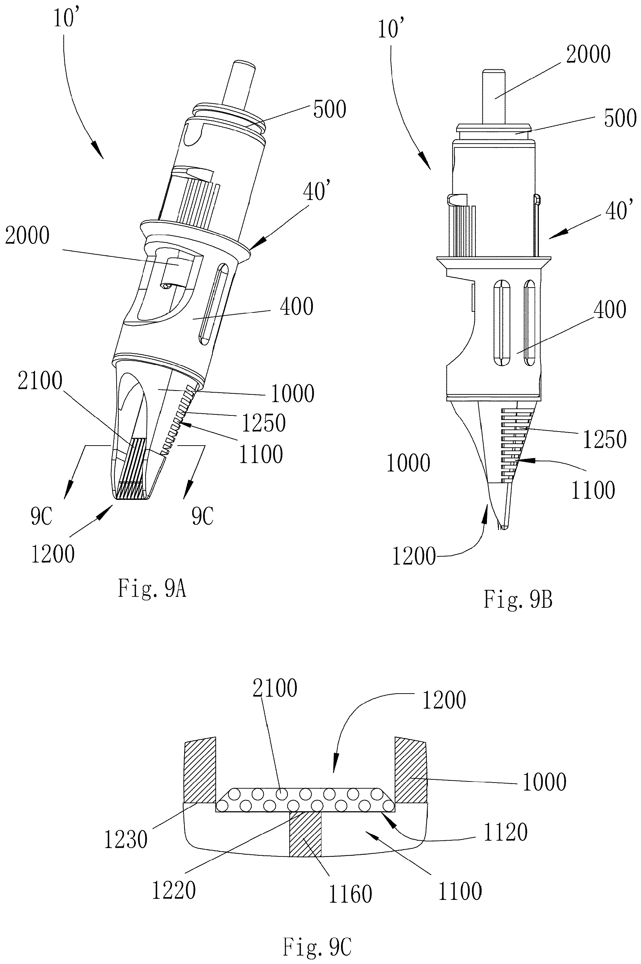

While the above examples are illustrated in the figures with a round tip portion or round needles and needle bundles, in different embodiments, the construction of the needle module may be readily modified or adapted to use flat or Magnum needles. In the latter case, the mouthpiece may typically have a generally rectangular mouth opening, as illustrated in FIGS. 9A, 9B and 9C. The example needle module 10' illustrated in these figures has a bundle of 15 needles arranged in two flat rows. The needles may be welded together. The needle module 10' includes a needle housing 40' for movably mounting a needle bundle 2000 therein. The needle housing 40' is formed by a tubular body portion 400, a mouthpiece 1000 and a cap 500. Housing 40' has a tubular longitudinal channel therein. The channel has an upper open end and a lower open end. The mouthpiece 1000 in needle module 10' has ink storage grooves 1100 between groove walls 1250 with contact opening 1120, an opening 1200 with a rectangular profile, and a guide surface 1220. Needle bundle tip portion 2100 is received in opening 1200 and contacts the guide surface 1220, under a biasing force as described earlier with regard to needle module 10. The ink storage grooves 1100 are separated by a reinforcing wall 1160 and extend from the reinforcing wall 1160 to end surfaces 1230.

As can be appreciated, a needle assembly described herein may be used or adapted to apply other types of liquids to skin. For example, the applied liquid may include colored liquids or pigments, or may include a medicinal or therapeutic agent, collagen, or other like or similar substances. The needle assembly may be used in a liquid applicator for applying the selected liquid.

Other features, modifications, and applications of the embodiments described here may be understood by those skilled in the art in view of the disclosure herein.

### CONCLUDING REMARKS

It will be understood that any range of values herein is intended to specifically include any intermediate value or sub-range within the given range, and all such intermediate values and sub-ranges are individually and specifically disclosed.

It will also be understood that the word "a" or "an" is intended to mean "one or more" or "at least one", and any singular form is intended to include plurals herein.

It will be further understood that the term "comprise", including any variation thereof, is intended to be open-ended and means "include, but not limited to," unless otherwise specifically indicated to the contrary.

When a list of items is given herein with an "or" before the last item, any one of the listed items or any suitable combination of two or more of the listed items may be selected and used.

Of course, the above described embodiments of the present disclosure are intended to be illustrative only and in no way limiting. The described embodiments are susceptible to many modifications of form, arrangement of parts, details and order of operation. The invention, rather, is intended to encompass all such modification within its scope, as defined by the claims.

## Claims

1. A needle assembly for a liquid applicator, comprising:
a housing comprising a tubular longitudinal channel and an inner surface, the channel comprising an upper open end and a lower open end, a plurality of liquid storage grooves for storing liquid therein by capillary action extending generally transversely in the inner surface above the lower open end;
a needle bundle mounted in the housing, the needle bundle comprising one or more needles longitudinally reciprocally movable in the channel and being biased against or towards the inner surface across the grooves such that, during longitudinal reciprocal movement of the needle bundle, (i) the needle bundle maintains contact with the inner surface and the liquid stored in the grooves or (ii) a capillary gap between the needle bundle and the inner surface is formed, for drawing the liquid out of the grooves by capillary action.

2. The needle assembly of claim 1, comprising a biasing member configured to bias the needle bundle to move longitudinally and against or towards the inner surface, wherein the biasing member optionally comprises a resilient band, which preferably comprises a silicone, latex, or rubber material.

3. The needle assembly of claim 1 or claim 2, wherein the inner surface is shaped to conform to an external profile of the needle bundle to increase contact area between the needle bundle and the liquid stored in the grooves, and wherein the inner surface is optionally a guide surface for guiding movement of the needle bundle.

4. The needle assembly of any one of claims 1 to 3, wherein the needle bundle comprises a plurality of needles welded to one another, and the needle bundle optionally comprises 1 to 27 needles.

5. The needle assembly of any one of claims 1 to 4, wherein the housing comprises a generally cylindrical or conical body having a circumference, and one or more of the grooves extend up to 120° along the circumference of the body.

6. The needle assembly of any one of claims 1 to 5, wherein the longitudinal channel has a generally circular profile or generally polygonal profile.

7. The needle assembly of any one of claims 1 to 6, wherein the grooves have a width of about 0.3 mm to about 0.6 mm.

8. The needle assembly of any one of claims 1 to 7, wherein the plurality of grooves comprises 3 to 12 grooves.

9. The needle assembly of any one of claims 1 to 8, wherein the housing comprises a generally conical lower portion and a generally cylindrical upper portion.

10. The needle assembly of any one of claims 1 to 9, wherein the housing comprises an observation window opposite the inner surface, and the needle bundle comprises a shaft portion and a needle tip portion, the shaft portion having a first longitudinal axis and the needle tip portion having a second longitudinal axis, the second axis being offset from the first axis towards the observation window.

11. The needle assembly of any one of claims 1 to 10, wherein the grooves are inter-connected.

12. The needle assembly of any one of claims 1 to 11, wherein the liquid applicator is an ink applicator and the liquid comprises ink.

13. A liquid applicator comprising the needle assembly of any one of claims 1 to 12.

14. The liquid applicator of claim 13, comprising a needle actuator for actuating downward movement of the needle bundle, and a handle coupling the needle actuator to the needle assembly.

15. A tattooing device comprising the needle assembly of any one of claims 1 to 12, or the liquid applicator of claim 13 or claim 14.
